# EUROPEAN PATENT SPECIFICATION

(11) **EP 3 142 662 B1**
(45) Date of publication and mention of the grant of the patent: **08.07.2020**
(21) Application number: 15739673.0
(22) Date of filing: 14.05.2015
(51) Int. Cl.: A61K 31/4015, A61P 31/00, A61P 25/02

(54) **PHARMACEUTICAL COMPOSITION FOR THE PREVENTION AND TREATMENT OF DISEASES ASSOCIATED WITH ELEVATED INDUCIBLE NITRIC OXIDE SYNTHASE**
PHARMAZEUTISCHE ZUSAMMENSETZUNGEN ZUR PRÄVENTION UND BEHANDLUNG VON ERKRANKUNGEN IM ZUSAMMENHANG MIT ERHÖHTER INDUZIERBARER STICKOXIDSYNTHASE
COMPOSITION PHARMACEUTIQUE POUR LA PRÉVENTION ET LE TRAITEMENT DE MALADIES ASSOCIÉES À LA SYNTHASE D'OXYDE NITRIQUE INDUCTIBLE ÉLEVÉE

(30) Priority: 14.05.2014 LV 143914
(43) Date of publication of application: 22.03.2017
(73) Proprietor: Akciju Sabiedriba "Olainfarm", 2114 Olaine (LV)
(72) Inventor: ZVEJNIECE, Liga, LV-1006 Riga (LV); DAMBROVA, Maija, LV-1006 Riga (LV); VEINBERGS, Grigorijs, LV-1006 Riga (LV); VORONA, Maksims, LV-1006 Riga (LV); KALVINS, Ivars, LV-1006 Riga (LV)
(74) Representative: Madgwick, Paul Roland
(86) International application number: PCT/IB2015/053554
(87) International publication number: WO 2015/173763

(56) References cited:
- WO-A1-2014/005721
- LAUREN BROOM ET AL: "Neuroprotection by the selective iNOS inhibitor GW274150 in a model of Parkinson disease", FREE RADICAL BIOLOGY AND MEDICINE, ELSEVIER INC, US, vol. 50, no. 5, 16 December 2010 (2010-12-16), pages 633-640, XP028137626, ISSN: 0891-5849, DOI: 10.1016/J.FREERADBIOMED.2010.12.026 [retrieved on 2010-12-23]
- LIGA ZVEJNIECE ET AL: "Investigation into Stereoselective Pharmacological Activity of Phenotropil", BASIC & CLINICAL PHARMACOLOGY & TOXICOLOGY, vol. 109, no. 5, 20 July 2011 (2011-07-20) , pages 407-412, XP55046454, ISSN: 1742-7835, DOI: 10.1111/j.1742-7843.2011.00742.x

## Description

### Field of invention

Present invention relates generally to the field of pharmacology and medicine. More specifically, the invention relates to the discovery of a second medical use of (4*R*)-2-(4-phenyl-2-oxopyrrolidin-1-yl)acetamide for the treatment and prevention of diseases caused by biological defense or NO excess, which are sepsis, and peripheral neuropathy.

### Summary of the invention

We have unexpectedly found that (4*R*)-2-(4-phenyl-2-oxopyrrolidin-1-yl)acetamide (I) can be successfully used for treatment of pathological conditions characterized by elevated expression of iNOS as it very effectively reduces overexpression of iNOS gene.

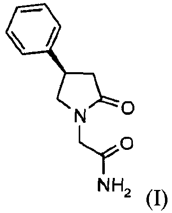

We have discovered that (4*R*)-2-(4-phenyl-2-oxopyrrolidin-1-yl)acetamide is highly effective in reduction of LPS-induced iNOS gene expression in mice brain tissue. This invention demonstrates that (4*R*)-2-(4-phenyl-2-oxopyrrolidin-1-yl)acetamide can be used as a new medication for the treatment of conditions characterized by elevated iNOS gene expression, for example, for endotoxin, cancer or cardiac transplantation induced sepsis.

We have also discovered that (4*R*)-2-(4-phenyl-2-oxopyrrolidin-1-yl)acetamide is highly effective in the treatment of peripheral neuropathy caused by formalin injection. This invention demonstrates that (4*R*)-2-(4-phenyl-2-oxopyrrolidin-1-yl)acetamide can be used as a new medication for the treatment of conditions characterized by elevated iNOS gene expression, for example, for treatment or prevention of peripheral neuropathy.

### Brief description of the figures

Fig. 1. Quantitative RT-PCR analysis of iNOS gene expression in brain tissue in LPS-induced septic shock model in mice, demonstrating a significant reduction of LPS-induced iNOS gene expression upon treatment with (4*R*)-2-(4-phenyl-2-oxopyrrolidin-1-yl)acetamide.
Fig. 2. Measurements of rectal temperature in LPS-induced septic shock model in mice, demonstrating a significant inhibition of LPS-induced hypothermia upon treatment with (4*R*)-2-(4-phenyl-2-oxopyrrolidin-1-yl)acetamide.
Fig. 3A, B. Results of the formalin-induced paw licking test (formalin-induced peripheral neuropathy), demonstrating a significant effect of (4*R*)-2-(4-phenyl-2-oxopyrrolidin-1-yl)acetamide in phase II of the said test.
Fig. 4A, B. Results of the formalin-induced paw licking test (formalin-induced peripheral neuropathy), demonstrating almost no effect of (4*R*,*S*)-2-(4-phenyl-2-oxopyrrolidin-1-yl)acetamide in phases I and II of the said test.

### Background of invention

iNOS is up-regulated under pathological conditions such as sepsis, hypertension, hypertrophy and heart failure (J. L. Balligand, D. Ungureanu-Longrois, W. W. Simmons et al., Journal of Biological Chemistry, 1994, vol. 269, no. 44, 27580-27588*;* K. Y. Xu, D. L. Huso, T. M. Dawson, D. S. Bredt, L. C. Becker, Proceedings of the National Academy of Sciences of the United States of America, 1999, vol. 96, no. 2, pp. 657-662). Sepsis is defined as the systemic response to infection, the most common cause being the contamination of the blood with bacteria. Septic shock (sepsis with hypotension) develops in almost half of the patients as a complication, with a mortality rate of 40-60%, despite treatment (M. A. Titheradge, Biochim. Biophys. Acta, 1999, 1411, 437-455). Sepsis is accompanied by alterations in several physiological parameters. These include a release of secondary messenger molecules like nitric oxide (NO), which amplify the immune response, resulting in metabolic changes. Additionally, cardiovascular abnormalities such as hypotension, peripheral vasodilatation associated with capillary leakage and low blood flow can cause inadequate tissue perfusion and multiple organ dysfunctions leading to high mortality (R. C. Bone, C. J. Grodzin, R. A. Balk. Chest, 1997, 112, 235-243*;* P. B. Corrêa, J. A. Pancoto, G. R. Oliveira-Pelegrin, E. C. Cárnio, M. J. Rocha, Journal of Neuroimmunology, 2007, 183, (1-2), 17-25). Evidence to date strongly suggests nitric oxide (NO) is the key mediator of the vasodilation and catecholamine-resistant hypotension that occurs in septic shock (Rees D. D., Biochem. Soc. Trans., 1995, 23, 1025-1029).

Sepsis is treated as a medical emergency with broad-spectrum antibiotics and fluids (0.9 % NaCl). Since all patients are different and the causes of sepsis are many, not every available treatment is right for each patient. Nevertheless, patients with severe sepsis or septic shock have a mortality rate of about 40-60 %, with the elderly having the highest death rates (C. R. Wira, K. Dodge, J. Sather, J. Dziura, West J. Emerg. Med., 2014, 15(1), 51-59). A 2006 study showed that the risk of death from sepsis increases by 7.6 % with every hour that passes before treatment begins (Kumar A., Roberts D., Wood K. E., Light B., Parrillo J. E., Sharma S., Suppes R., Feinstein D., Zanotti S., Taiberg L., Gurka D., Kumar A., Cheang M., Crit. Care Med., 2006, 34(6), 1589-96). Thus, indicating the lack of effectiveness of current therapies and the need for new drug targets.

Recent studies have shown promising efficacy of NOS inhibitors in sepsis models. A multicenter, randomized, placebo-controlled double-blind study of the non-selective NOS inhibitor Nω-nitro-L-arginine methyl ester (L-NAME) was carried out in patients with septic shock. Results of the phase II trial were promising: a decrease in plasma nitrate levels in the treatment group was associated with a decreased requirement for vasopressors, earlier shock resolution and an improvement in vascular resistance (Bakker J., Grover R., McLuckie A., Holzapfel L., Andersson J., Lodato R., Watson D., Grossman S., Donaldson J., Takala J., Crit. Care Med., 2004, 32(1), 1-12*;* Watson D., Grover R., Anzueto A., Lorente J., Smithies M., Bellomo R., Guntupalli K., Grossman S., Donaldson J., Le Gall J. R., Crit. Care Med., 2004, 32(1), 13-20). However, the phase III trial was terminated prematurely due to an increase in mortality in the treatment group, with a higher proportion of deaths from cardiac dysfunction related to pulmonary hypertension (E. Abraham, M. Singer, Mechanisms of Sepsis-Induced Organ Dysfunction and Recovery. Springer, 2007*).*

It was shown that NO can also stimulate tumor growth and metastasis by promoting migratory, invasive, and angiogenic abilities of tumor cells, which may also be triggered by activation of cyclo-oxygenase (COX)-2. Thus, selective inhibitors of NOS, COX, or both may have a therapeutic role in certain cancers (Lala P. K, Chakraborty C., Lancet Oncol., 2001, 2(3), 149-56). Selective expression of iNOS in liver causes hepatic insulin resistance along with deranged insulin signaling, leading to hyperglycemia and hyperinsulinemia (Shinozaki S.L., Choi C. S., Shimizu N., Yamada M., Kim M., Zhang T., Shiota G., Dong H. H., Kim Y. B., Kaneki M., J. Biol. Chem., 2011, 286(40), 34959-75) and expression of recombinant iNOS in cerebral arteries reduces vasomotor reactivity to both vasoconstrictor and vasodilator agonists (Eguchi D.L., D'Uscio L. V., Wambi C., Weiler D., Kovesdi I., O'Brien T., Katusic Z. S., Am. J. Physiol. Heart Circ. Physiol., 2002, 283(6), H2560-6). iNOS could be an important mediator in coeliac disease (I. Daniels, D. Cavill, I. A. Murray, R. G. Long, Clinica Chimica Acta, 2005, Volume 356, Issues 1-2, 134-142). Moreover, it has been shown that iNOS is elevated after haemorrhage, and that iNOS-derived NO participates in the lung and liver injury post-resuscitation from shock. Inhibition of iNOS with the selective inhibitor N6-(iminoethyl)-L-lysine resulted in a marked reduction of liver injury following hemeorragic shock (Tipoe G. L., Leung T. M., Liong E., So H., Leung K. M., Lau T. Y., Tom W. M., Fung M. L., Fan S. T., Nanji A. A. Histol. Histopathol., 2006, 21(11), 1157-65).

Synthesis and release of nitric oxide (NO) via the enzyme inducible nitric oxide synthase (iNOS) from astrocytes and microglia is a way in which neuroinflammation directly influences neuronal apoptosis. NO plays a critical role in brain function in general and is believed to have an important role in numerous neuropathological processes, including trauma, Alzheimer's disease, and cerebral ischemia (R. S. Clark, P. M. Kochanek, M. A. Schwarz, J. K. Schiding, D. S. Turner, M. Chen, T. M. Carlos, S. C. Watkins, Pediatr. Res., 1996, 39, 784*;* Y. Vodovotz, M. S. Lucia, K. C. Flanders, L. Chesler, Q. W. Xie, T. W. Smith, J. Weidner, R. Mumford, R. Webber, C. Nathan, et al., J. Exp. Med., 1996, 184, 1425-33*;* K. M. Boje, P. K. Arora, Brain Res., 1992, 587, 250-6). There is now a substantial body of evidence to support that nitric oxide (NO) is an intercellular messenger molecule that is thought to be involved in synaptic transmission in both the central and peripheral nervous systems (J. Garthwaite, S. L. Charles, R. Chess-WilliamsNature, 1988, 336, 385-388*;* D. S. Bredt, S. H. Snyder, Neuron, 1992, 8, 3-11). In the spinal cord, NO has been demonstrated to play a role in modulation of nociceptive transmission and plasticity. The neuronal NO synthase is concentrated in the superficial dorsal horn of the spinal cord (N. J. Dun, S. L. Dun, S. Y. Wu, U. Forstermann, H. H. Schmidt, L. F. Tseng, Neuroscience, 1993, 54, 845-857*;* G. Terenghi, V. Riveros-Moreno, L. D. Hudson, N. B. Ibrahim, J. M. PolakJ. Neurol. Sci., 1993, 118,34-37*;* S. Saito, G. J. Kidd, B. D. Trapp, T. M. Dawson, D. S. Bredt, D. A. Wilson, R. J. Traystman, S. H. Snyder, D. F. Hanley, Neuroscience, 1994, 59, 447-456). Formalin injection produced long-lasting increase of NO synthase expression in the spinal cord (T. Herdegen, S. Rudiger, B. Mayer, R. Bravo, M. Zimmermann, Mol. Brain Res., 1994, 22, 245-258*;* H. H. Lam, D. F. Hanley, B. D. Trapp, S. Saito, S. Raja, T. M. Dawson, H. Yamaguchi, Neurosci. Lett., 1996, 210, 201-204). Systemic and intrathecal administration of NO synthase inhibitors such as L-NAME or 7-nitroindazole (7-NI) reduces nociceptive responses to formalin-produced peripheral neuropathy in mice and rats (P. K. Moore, R. C. Babbedge, P. Wallace, Z. A. Gaffen, S. L. Hart, Br. J. Pharmacol., 1993, 108, 296-297*;* A. K. Roche, M. Cook, G. L. Wilcox, K. C. Kajander, Pain, 1996, 66, 331-341). NO donors applied intrathecally not only cause a reduction in tail flick or paw withdrawal latency (T. Inoue, T. Mashimo, S. Shibuta, I. Yoshiya, J. Neurol. Sci., 1997, 153, 1-7*;* Y.-X. Tao, R. A. Johns, Eur. J. Pharmacol., 2000, 392, 141-145), but also facilitate formalin-induced nociceptive behaviors in the second phase (S. Shibuta, T. Mashimo, P. Zhang, A. Ohara, I. Yoshiya, J. Neurol. Sci., 1996, 141, 1-5). In addition, injection of formalin into a hind paw evokes a biphasic spinal release of NO metabolites (K. Okuda, C. Sakurada, M. Takahashi, T. Yamada, T. Sakurada, Pain, 2001, 92, 107-115). These results indicate that NO is involved in the central mechanism of peripheral neuropathies induced hyperalgesia at the spinal cord level.

WO 2014/005721 A1 discloses the use of (4R)-2-oxo-4-phenyl-1pyrrolidineacetamide (i.e., (R)-phenylpiracetam) in the treatment of Parkinson's disease, a disease which is characterized by elevated expression of iNOS.

It was shown that selective iNOS inhibitors such as aminoguanidine hydrochloride and AR-C102222 reduce peripheral nociception in formalin-induced paw licking test II phase *(*S. P. Dudhgaonkar, D. Kumar, A. Naik, A. R. Devi, D. U. Bawankule, S. K Tandan, European Journal of Pharmacology, 2004, 492, 2-3, 117-122*;* C. J. LaBuda, M Koblish, P. Tuthill, R. E. Dolle, P. J. Little. European Journal of Pain, 2006, 10, (6), 505-512*.).* In II phase of formalin-induced paw licking test anti-nociceptive activity show drugs, which are used to treat peripheral neuropathy, for example gabapentin, pregabalin and amitriptyline *(*H. Gustafsson, K. Flood, O. G. Berge, E. Brodin, L. Olgart, C. O. Stiller. Experimental Neurology, 2003, 182, (2), 427-434*;* H. Gustafsson, J. Sandin. European Journal of Pharmacology, 2009, Volume 605, 1-3, 103-108*;* J Sawynok, A. Reid Pain, 2001, 93, 1, pages 51-59*).* Peripheral neuropathy is associated with excessive neurodegenerative processes in both peripheral nervous system and CNS which contributes to the initiation and maintenance of persistent pain *(*Ellis A., Bennett D. L., Br. J. Anaesth., 2013, Jul; 111 (1), 26-37*).* Peripheral neuropathy, a result of nerve damage, in patients often causes weakness, numbness and pain, usually in hands and feet, but it may also occur in other areas of the body. It is thought to be due to pathologic changes in, or damage to, neurons in the peripheral or central nervous system *(*Freynhagen R., Bennett M I, BMJ, 2009, 339, b3002*).* There are some medications available for the treatment of peripheral neuropathy; however, many patients do not achieve a satisfactory response or experience serious side effects. Such drugs as tricyclic antidepressants, SNRI, anticonvulsants and opioid analgesics can be used for treatment of peripheral neuropathy, but they do not bring about the full improvement of life quality for the patients due to serious side effects and moderate clinical effectiveness (Table 1). The discovery of pharmaceuticals abating and preventing the peripheral neuropathy more effective than existing drugs is of substantial importance.

**Table 1. Medications to treat peripheral neuropathy.**

| **Medication** | **Side effects** | **Advantages** | **Disadvantages** |
|---|---|---|---|
| Amitriptyline (tricyclic antidepressants) | Anticholinergic side effects, sedation, cardiotoxicity, reduced seizure threshold | Well-established efficacy, once daily dosing, inexpensive, helpful for sleep disturbances | Often poorly tolerated, especially in elderly; high risk in overdose; higher dose needed to treat depression |
| Duloxetine (SNRI*) | Nausea, increased BP* (not clinically significant), rare hepatoxicity | No anticholinergic side effects or cardiac toxicity, easy titration, once daily dosing, same dose is effective for depression, anxiety, and neuropathic pain, FDA* approved for painful diabetic neuropathy | Expensive, limited long-term experience in neuropathic pain |
| Venlafaxine (SNRI) | GI side effect, increased HR* and BP, ECG* abnormalities (rare) | No anticholinergic side effects, once daily dosing (with XR form), same dose is effective for depression, anxiety, and neuropathic pain | Expensive, limited long-term experience in neuropathic pain |
| Gabapentin (anticonvulsant) | Dizziness, somnolence, headache, diarrhea, confusion, nausea, peripheral edema, weight gain | No anticholinergic side effects, no hepatic metabolism, no cardiac toxicity | Slow titration, 3 times per day dosing, nonlinear kinetics |
| Pregabalin (anticonvulsant) | Somnolence, dizziness, edema, weight gain | Faster, simpler titration compared with gabapentin, FDA-approved for painful diabetic neuropathy | Expensive, Schedule V controlled substance, twice daily dosing |
| Topical Lidocaine (local anesthetic) | Application site irritation | Negligible systemic absorption | Effective only for localized pain, expensive |
| Methadone (opioid analgesic) | Sedation, constipation, nausea, vomiting, dry mouth, hypogonadism, immunologic changes, hyperalgesia, increased risk of falls and cognitive problems in the elderly | Quick onset, effective for many different types of pain, good efficacy | Potential for abuse/misuse, limited evidence for long-term safety in neuropathic pain |
| Tramadol (opioid analgesic) | Nausea, vomiting, sweating, dry mouth, dizziness, sedation, decreased seizure threshold | Quick onset of pain relief, less abuse potential than strong opioids, long-acting formulation available, helpful for many different types of pain | Risk of serotonin syndrome, limited long-term evidence, some risk of abuse/misuse |

| | | | |
|---|---|---|---|
| *SNRI - Serotonin-norepinephrine reuptake inhibitor; BP - blood pressure; ECG - electrocardiogram; FDA - Food & Drug Administration; GI - gastrointestinal; HR - heart rate. | | | |

Therefore, search for effective and safe iNOS inhibitors is of substantial importance.

### Description of invention

According to the current invention, (4*R*)-2-(4-phenyl-2-oxopyrrolidin-1-yl)acetamide (CAS No. 949925-07-9) can be used for treatment of conditions characterized by elevated expression of iNOS because it very effectively reduces overexpression of iNOS gene. It was completely unexpected because racemic (4*R*,*S*)-2-(4-phenyl-2-oxopyrrolidin-1-yl)acetamide (CAS No. 77472-70-9) is used in human medicine as antidepressant, stress protective agent and modulator of locomotor activity (D. Mashkovsky. "Medicinal drugs", Moscow, MEDITSINA Publishers, 1988; part I, pp. 70-79, 122-123*)* and enantiomerically pure (4*R*)-2-(4-phenyl-2-oxopyrrolidin-1-yl)acetamide (CAS No. 949925-07-9) was disclosed for use as antidepressant, stress-protective agent, modulator of locomotor activity, muscle relaxant and analgesic (WO2007104780).

We have discovered that (4*R*)-2-(4-phenyl-2-oxopyrrolidin-1-yl)acetamide is highly effective in reduction of LPS-induced iNOS gene expression in mice brain tissue, while (4*S*)-2-(4-phenyl-2-oxopyrrolidin-1-yl)acetamide is absolutely inactive and (4*R*,*S*)-2-(4-phenyl-2-oxopyrrolidin-1-yl)acetamide demonstrates tendency to reduce LPS action. In this respect a pure *R*-enantiomer has an unexpected and relevant preference against both racemate and *S-*enantiomer. This invention demonstrates that (4*R*)-2-(4-phenyl-2-oxopyrrolidin-1-yl)acetamide can be used as a new medication for the treatment of conditions characterized by elevated iNOS gene expression, for example, for endotoxin, cancer or cardiac transplantation induced sepsis.

We have discovered that (4*R*)-2-(4-phenyl-2-oxopyrrolidin-1-yl)acetamide is highly effective in the treatment of peripheral neuropathy caused by formalin injection. We have discovered also that action of (4*R*)-2-(4-phenyl-2-oxopyrrolidin-1-yl)acetamide is based on the surprising and unexpected reduction of LPS-induced iNOS gene expression in mice brain tissue. This invention demonstrate that (4*R*)-2-(4-phenyl-2-oxopyrrolidin-1-yl)acetamide can be used as a new medication for the treatment of conditions characterized by elevated iNOS gene expression, for example, for treatment or prevention of peripheral neuropathy.

It is known that (4*R*)-2-(4-phenyl-2-oxopyrrolidin-1-yl)acetamide does not cause side effects on the body system up till the dose of 250 mg/kg after i.p. administration (Zvejniece L, Svalbe B, Veinberg G, Grinberga S, Vorona M, Kalvinsh I, Dambrova M. Basic Clin. Pharmacol. Toxicol., 2011, 109 (5), 407-12*).* (4*R*)-2-(4-phenyl-2-oxopyrrolidin-1-yl)acetamide is totally excreted from body in unmetabolized form. Therefore, (4*R*)-2-(4-phenyl-2-oxopyrrolidin-1-yl)acetamide is promising drug for the treatment of sepsis and other conditions with elevated level of iNOS synthase, without severe and life-threatening side effects.

The effectiveness of (4*R*)-2-(4-phenyl-2-oxopyrrolidin-1-yl)acetamide in the treatment of pathological conditions characterized by elevated level of iNOS, for example by sepsis and peripheral neuropathy, was strictly confirmed by experimental results of tests aimed at the evaluation of:
a) LPS injection induced iNOS gene overexpression and hypothermia in animals acute septic shock model (M. A. Titheradge, Biochim. Biophys. Acta, 1999, 1411, 437-455);
b) behavioral response of animals in formalin-induced paw licking test (Carter M., Shieh J. C. Guide to Research Techniques in Neuroscience, 2010, Burlington, MA: Academic Press, pp. 51-2).

It is established that bacterial endotoxin, a lipopolysaccharide (LPS) component of the outer membrane of Gram-negative bacteria, is the major mediator of the high morbidity and mortality rates characteristic of Gram-negative septic shock. Administration of endotoxin experimentally to animals mimics the symptoms of septic shock. The injection of LPS increase the expression of inducible NO synthase (iNOS), whereas elevated expression of iNOS results in sustained production of large quantities of NO which are postulated to mediate the alterations in the vascular system and the tissue damage apparent in septic shock and multiple organ dysfunction syndrome (M. A. Titheradge, Biochim. Biophys. Acta, 1999, 1411, 437-455). During endotoxaemia iNOS expression and increases in iNOS mRNA and protein have been observed in a variety of cell types including endothelial cells, macrophages, Kupffer cells, hepatocytes, vascular smooth muscle cells, kidney cells, chondrocytes, cardiac myocytes, pancreatic islets and fibroblasts (S. M. Morris, T. R. Billiar. Am. J. Physiol., 1994, 266, 829-839*;* C. Nathan. FASEB J., 1992, 6, 3051-3064*;* M. Salter, R. G. Knowles, S. Moncada. FEBS Lett., 1991, 291, 145-149).

In mammals, LPS injection induces hypothermia by reducing metabolic heat production and by an induction of cold-seeking behavior, even though their body temperature is decreasing (A. A. Romanovsky, M. C. Almeida, D. M. Aronoff, A. L. Ivanov, J. P. Konsman, A. A. Steiner, V. F. Turek, Frontiers in Bioscience, 2005, 10, pp. 2193-2216*;* A. Y. Rudaya, A. A. Steiner, J. R. Robbins, A. S. Dragic, A. A. Romanovsky, American Journal of Physiology, Regulatory, Integrative and Comparative Physiology, 2005, 289 (5), pp. R1244-R1252). It is fundamentally accepted that LPS-induced hypothermia, as a sign of septic shock, is a brain mediated response and is triggered by release of free radicals like NO and cryogenic cytokines from LPS-processing organs, which is potentially lethal (R. S. Saia, E. C. Carnio. Life Sciences, 2006, 79 (15), pp. 1473-1478*;* M. J. Kluger, Physiological Reviews, 1991, 71 (1), pp. 93-127).

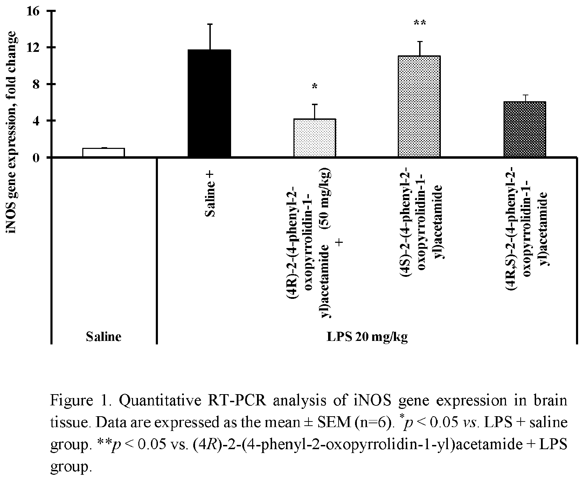

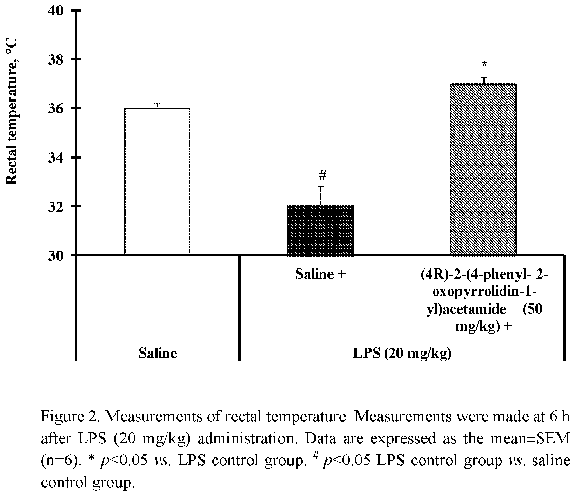

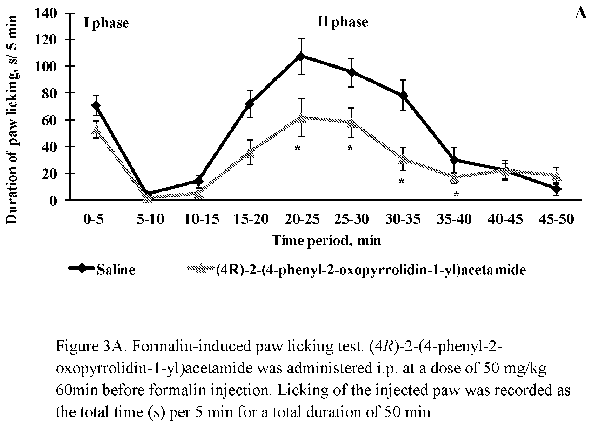

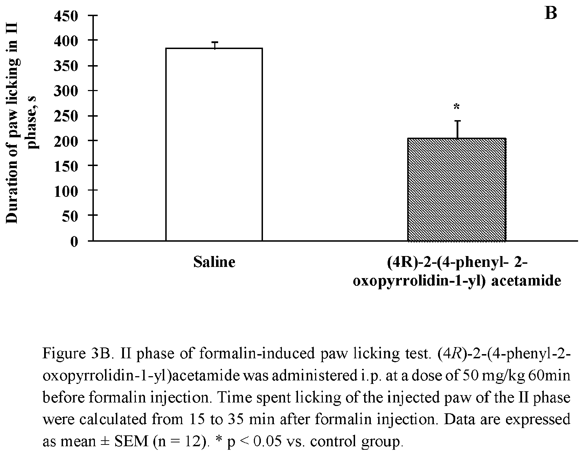

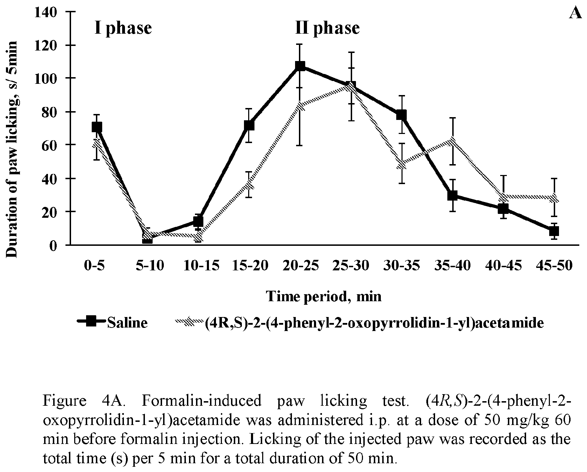

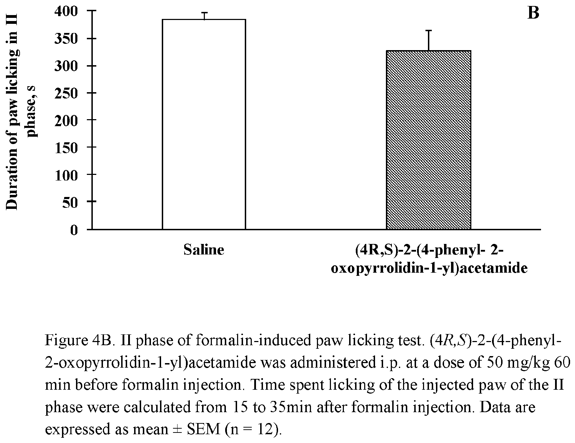

Results presented in Fig. 1 and Fig. 2 show that (4*R*)-2-(4-phenyl-2-oxopyrrolidin-1-yl)acetamide when administered together with LPS effectively reduced the expression of iNOS gene and inhibited the LPS-induced hypothermia in mice. At the same time, (4*R,S*)-2-(4-phenyl-2-oxopyrrolidin-1-yl)acetamide and (4*S*)-2-(4-phenyl-2-oxopyrrolidin-1-yl)acetamide did not prevent the LPS-induced overexpression of iNOS gene. In addition, (4*R*)-2-(4-phenyl-2-oxopyrrolidin-1-yl)acetamide reduced responses in the formalin-induced paw licking test in vivo (Fig. 3A, 3B), at the same time racemic (4*R*,*S*)-2-(4-phenyl-2-oxopyrrolidin-1-yl)acetamide was ineffective (Fig. 4A, 4B).

Obtained experimental data evidence about significant difference in ability of (4*R*)-2-(4-phenyl-2-oxopyrrolidin-1-yl)acetamide to depress iNOS gene expression in comparison to (4*S*)-2-(4-phenyl-2-oxopyrrolidin-1-yl)acetamide. These data speaks for efficacy of (4*R*)-2-(4-phenyl-2-oxopyrrolidin-1-yl)acetamide in the treatment of pathological conditions characterized by substantially elevated iNOS gene expression, such as bacterial LPS induced hypothermia and septic shock. This invention also brings evidence that pharmaceutical compositions based on (4*R*)-2-(4-phenyl-2-oxopyrrolidin-1-yl)acetamide but not the (4*S*)-2-(4-phenyl-2-oxopyrrolidin-1-yl)acetamide could be used as an appropriate medicine for treatment of other diseases and conditions with pathologically elevated levels of iNOS, such as peripheral neuropathies.

### Pharmaceutical compositions

(4*R*)-2-(4-phenyl-2-oxopyrrolidin-1-yl)acetamide may be used according to this invention in pharmaceutical compositions in the form of any pharmaceutically acceptable salts, solvates, conjugates, hydrates, solvates, co-crystals and polymorphs. Any references to (4*R*)-2-(4-phenyl-2-oxopyrrolidin-1-yl)acetamide in this description should be understood as also referring to such salts, solvates, conjugates, hydrates, solvates, co-crystals and polymorphs.

(4*R*)-2-(4-phenyl-2-oxopyrrolidin-1-yl)acetamide can be used in pharmaceutical compositions or preparations containing the compound in combination with a pharmaceutically acceptable carrier. The term "carrier" applied to pharmaceutical compositions of the invention refers to a diluent, excipient, or vehicle with which an active compound is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington's Pharmaceutical Sciences" by E.W. Martin, 18th Edition.

Suitable pharmaceutically acceptable carriers include inert solid fillers or diluents and sterile aqueous or organic solutions. Thus, for oral administration the compounds can be combined with a suitable solid or liquid carrier or diluents to form capsules, tablets, powders, syrups, solutions, suspensions and the like. The pharmaceutical compositions may, if desired, contain additional components such as flavorings, sweeteners, excipients and the like. For parenteral administration the compounds can be combined with sterile aqueous or organic media to form injectable solutions or suspensions. The injectable solutions can then be administered intravenously, intraperitoneally, subcutaneously, or intramuscularly.

The active ingredients of the invention, together with one or more conventional adjuvants, carriers, or diluents, may be placed into the form of pharmaceutical compositions and unit dosages thereof, and in such form may be employed as solids, such as coated or uncoated tablets or filled capsules, or liquids, such as solutions, suspensions, emulsions, elixirs, or capsules filled with the same, all for oral use; in the form of suppositories or capsules for rectal administration or in the form of sterile injectable solutions for parenteral (including intravenous or subcutaneous) use. Such pharmaceutical compositions and unit dosage forms thereof may comprise conventional or new ingredients in conventional or special proportions, with or without additional active compounds or principles, arid such unit dosage forms may contain any suitable effective amount of the active ingredient commensurate with the intended daily dosage range to be employed.

For oral administration in the form of a tablet or capsule, the active drug component can be combined with a non-toxic, pharmaceutically acceptable excipients such as binding agents *(e.g.,* pregelatinized maize starch, polyvinylpyrrolidone or hydroxypropyl methylcelluiose); fillers *(e.g.,* lactose, sucrose, glucose, mannitol, sorbitol and other reducing and non-reducing sugars, microcrystalline cellulose, calcium sulfate, or calcium hydrogen phosphate); lubricants *(e.g.,* magnesium stearate, talc, or silica, steric acid, sodium stearyl fumarate, glyceryl behenate, calcium stearate, and the like); disintegrants (e.g., potato starch or sodium starch glycolate); or wetting agents (e.g., sodium lauryl sulphate), coloring and flavoring agents, gelatin, sweeteners, natural and synthetic gums (such as acacia, tragacanth or 5 alginates), buffer salts, carboxymethyicellulose, polyethyleneglycol, waxes, and the like. For oral administration in liquid form, the drug components can be combined with non-toxic, pharmaceutically acceptable inert carriers (e.g., ethanol, glycerol, water), suspending agents (e.g., sorbitol syrup, cellulose derivatives or hydrogenated edible fats), emulsifying agents (e.g., lecithin or acacia), nonaqueous vehicles (e.g., almond oil, oily esters, ethyl alcohol or fractionated vegetable oils), preservatives (e.g., methyl or propyl-p-hydroxybenzoates or sorbic acid), and the like. Stabilizing agents such as antioxidants (BHA, BHT, propyl gallate, sodium ascorbate and citric acid) can also be added to stabilize the dosage forms. The tablets can be coated by methods well known in the art.

Use of the compounds of the present invention in the manufacture of a medicament for treatment of a living animal for suppressing of abnormal iNOS gene expression is carried out in the usual manner comprising the step of admixing an effective amount of a compound of the invention with a pharmaceutically-acceptable diluent, excipient, or carrier, and the method-of-treating, pharmaceutical compositions, and use of a compound of the present invention in the manufacture of a medicament.

### Methods of administration and dosage forms

Due to their high degree of activity and their low toxicity, together presenting a most favorable therapeutic index, the active principles of the invention may be administered to a subject, e.g., a living animal (including a human) body, in need thereof, for the treatment, alleviation, modulation, amelioration, palliation, or elimination of an indication or condition which is susceptible thereto, or representatively of an indication or condition set forth elsewhere in this application, optionally concurrently, simultaneously, or together with one or more pharmaceutically-acceptable excipients, carriers, or diluents, and optionally in the form of a pharmaceutical composition thereof, whether by oral, rectal, or parental (including intravenous and subcutaneous) or in some cases even topical route, in an effective amount. Suitable dosage ranges are 1-1000 milligrams daily, 10-500 milligrams daily, and 50-250 milligrams daily, depending as usual upon the exact mode of administration, form in which administered, the indication toward which the administration is directed, the subject involved and the body weight of the subject involved, and the preference and experience of the physician or veterinarian in charge. The term "therapeutically effective" applied to dose or amount refers to that quantity of a compound or pharmaceutical composition that is sufficient to result in a desired activity upon administration to a living animal body in need thereof. The active agents of the present invention may be administered orally, topically, parenterals, or mucosally *(e.g.,* buccally, by inhalation, or rectally) in dosage unit formulations containing conventional non-toxic pharmaceutically acceptable carriers. It is usually desirable to use the oral route. The active agents may be administered orally in the form of a capsule, a tablet, or the like *(see* Remington: The Science and Practice of Pharmacy, 20th Edition (2000), Philadelphia, PA). The orally administered medicaments may be administered in the form of a time-controlled release vehicle, including diffusion-controlled systems, osmotic devices, dissolution controlled matrices, and erodible/degradable matrices.

The formulations of the invention can be delivered parenterally, *i.e.,* by intravenous (i.v.), intracerebroventricular (i.c.v.), subcutaneous (s.c), intraperitoneal (i.p.), intramuscular (i.m.), subdermal (s.d.), or intradermal (i.d.) administration, by direct injection, via, for example, bolus injection or continuous infusion. Formulations for injection can be presented in unit dosage form, *e.g.,* in ampoules or in multi-dose containers, with an added preservative. The compositions can take such forms as excipients, suspensions, solutions, or emulsions in oily or aqueous vehicles, and can contain formulatory agents such as suspending, stabilizing and/or dispersing agents. Alternatively, the active ingredient can be in powder form for reconstitution with a suitable vehicle, e.g., sterile pyrogen-free water, before use.

Compositions of the present invention can also be formulated for rectal administration, *e.g.,* as suppositories or retention enemas *(e.g.,* containing conventional suppository bases such as cocoa butter or other glycerides).

The compositions of the invention can be also introduced in microspheres or microcapsules, e.g., fabricated from polyglycolic acid/lactic acid (PGLA).

Liquid preparations for oral administration can take the form of, for example, solutions, syrups, emulsions or suspensions, or they can be presented as a dry product for reconstitution with water or other suitable vehicle before use.

Preparations for oral administration can be suitably formulated to give controlled or postponed release of the active compound. The active drugs can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines, as is well known.

Drugs of the invention may also be delivered by the use of monoclonal antibodies as individual carriers to which the compound molecules are coupled.

Active drugs may also be coupled with soluble polymers as targetable drug carriers. Such polymers can include polyvinylpyrrolidone, pyran copolymer, polyhydroxy-propyl methacrylamide-phenol, polyhydroxy-ethyl-aspartamide-phenol, or polyethyleneoxide-polylysine substituted with palmitoyl residues.

Furthermore, active drug may be coupled to a class of biodegradable polymers useful in achieving controlled release of a drug, for example, polylactic acid, polyglycolic acid, copolymers of polylactic and polyglycolic acid, polyepsilon caprolactone, polyhydroxybutyric acid, polyorthoesters, polyacetals, polyhydropyrans, polycyanoacrylates, and cross-linked or amphipathic block copolymers of hydrogels.

For administration by inhalation, the therapeutics according to the present invention can be conveniently delivered in the form of an aerosol spray presentation from pressurized packs or a nebulizer, with the use of a suitable propellant, *e.g.,* dichlorodifluoromethane, trichlorofluoromethane, dichlorotetrafluoroethane, carbon dioxide, or other suitable gas. In the case of a pressurized aerosol, the dosage unit can be determined by providing a valve to deliver a metered amount. Capsules and cartridges of, *e.g.,* gelatin for use in an inhaler or insufflators can be formulated containing a powder mix- of the compound and a suitable powder base such as lactose or starch.

The compositions may, if desired, be presented in a pack or dispenser device which may contain one or more unit dosage forms containing the active ingredient, optionally at various dosage levels to act as a titration pack. The pack may, for example, comprise metal or plastic foil, such as a blister pack.

As disclosed herein, the dose of the components in the compositions of the present invention is determined to ensure that the dose administered continuously or intermittently will not exceed an amount determined after consideration of the results in test animals and the individual conditions of a patient.

A specific dose naturally varies depending on the dosage procedure, the conditions of a patient or a subject animal such as age, body weight, sex, sensitivity, feed, dosage period, drugs used in combination, seriousness of the disease. The appropriate dose and dosage times under certain conditions can be determined by the test based on the above-described indices but may be refined and ultimately decided according to the judgment of the practitioner and each patient's circumstances (age, general condition, severity of symptoms, sex, etc.) according to standard clinical techniques.

Toxicity and therapeutic efficacy of the compositions of the invention can be determined by standard pharmaceutical procedures in experimental animals, *e.g.,* by determining the LD₅o (the dose lethal to 50 % of the population) and the ED₅₀ (the dose therapeutically effective in 50 % of the population). The dose ratio between therapeutic and toxic effects is the therapeutic index and it can be expressed as the ratio ED₅o/LD₅₀. Compositions that exhibit large therapeutic indices are preferred.

### Representative pharmaceutical compositions

With the aid of commonly used solvents, auxiliary agents and carriers, the reaction products can be processed into tablets, coated tablets, capsules, drip solutions, suppositories, injection and infusion preparations, and the like and can be therapeutically applied by the oral, rectal, parenteral, and additional routes. Representative non-limiting pharmaceutical compositions follow.
(a) Tablets suitable for oral administration which contain the active ingredient may be prepared by conventional tableting techniques.
(b) For suppositories, any usual suppository base may be employed for incorporation thereinto by usual procedure of the active ingredient, such as a polyethylene glycol which is a solid at normal room temperature but which melts at or about body temperature.
(c) For parental (including intravenous and subcutaneous) sterile solutions, the active ingredient together with conventional ingredients in usual amounts are employed, such as for example sodium chloride and double-distilled water q.s., according to conventional procedure, such as filtration, aseptic filling into ampoules or IV-drip bottles, and autoclaving for sterility.
Other suitable pharmaceutical compositions will be immediately apparent to one skilled in the art.

### Exemplary formulations

The following exemplary formulations are given by way of illustration only and are not to be construed as limiting this invention in any way.

### Formulation 1. Tablet

A suitable formulation for a tablet containing 10 milligrams of active ingredient is as follows:

| | |
|---|---|
| Active Ingredient | 10 mg |
| Lactose | 61 mg |
| Microcrystalline Cellulose | 25 mg |
| Talcum | 2 mg |
| Magnesium stearate | 1 mg |
| Colloidal silicon dioxide | 1 mg |

### Formulation 2. Coated tablet

Another suitable formulation for a tablet containing 100 mg is as follows:

| | |
|---|---|
| Active Ingredient | 100 mg |
| Polyvinylpyrrolidone, crosslinked | 10 mg |
| Potato starch | 20 mg |
| Polyvinylpyrrolidone | 19 mg |
| Magnesium stearate | 1 mg |
| Microcrystalline Cellulose | 50 mg |

Film coated and colored. The film coating material consists of:

| | |
|---|---|
| Hypromellose | 10 mg |
| Microcryst. Cellulose | 5 mg |
| Talcum | 5 mg |
| Polyethylene glycol | 2 mg |
| Color pigments | 5 mg |

### Formulation 3. Capsule

A suitable formulation for a capsule containing 50 milligrams of active ingredient is as follows:

| | |
|---|---|
| Active Ingredient | 50 mg |
| Corn starch | 26 mg |
| Dibasic calcium phosphate | 50 mg |
| Talcum | 2 mg |
| Colloidal silicon dioxide | 2 mg |

Filled in a gelatin capsule.

### Formulation 4. Solution for injection

A suitable formulation for an injectable solution is as follows:

| | |
|---|---|
| Active Ingredient | 10 mg |
| Sodium chloride | q.s. |
| Water for Inj ection | 1.0 mL |

### Formulation 5. Liquid oral solution

A suitable formulation for 1 liter of an oral solution containing 200 milligrams of active ingredient in one milliliter of the mixture is as follows:

| | |
|---|---|
| Active Ingredient | 200 mg |
| Saccharose | 250 mg |
| Glucose | 300 mg |
| Sorbitol | 150 mg |
| Orange flavor | 10 mg |
| Colorant | q.s. |
| Purified water | add 100 mL |

### Formulation 6. Liquid oral solution

Another suitable formulation for 1 liter of a liquid mixture containing 500 milligrams of active ingredient in one milliliter of the mixture is as follows:

| | |
|---|---|
| Active Ingredient | 500 mg |
| Saccharose | 750 mg |
| Glucose | 300 mg |
| Sorbitol | 150 mg |
| Orange flavor | 10 mg |
| Colorant | q.s. |
| Purified water | add 100 mL |

### Formulation 7. Aerosol solution

180 g of aerosol solution contains:

| | |
|---|---|
| Active Ingredient | 10 g |
| Oleic acid | 5 g |
| Ethanol | 81 g |
| Purified Water | 9 g |
| Tetrafluoroethane | 75 g |

15 ml of the solution are filled into aluminum aerosol cans, capped with a dosing valve, purged with 3.0 bar.

### Formulation 8. TD.S.solution

100 g of solution contains:

| | |
|---|---|
| Active Ingredient | 10.0 g |
| Ethanol | 57.5 g |
| Propyleneglycol | 7.5 g |
| Dimethylsulfoxide | 5.0 g |
| Hydroxyethylcellulose | 0.4 g |
| Purified water | 19.6 g |

1.8 ml of the solution are placed on a fleece covered by an adhesive backing foil. The system is closed by a protective liner which will be removed before use.

### Formulation 9. Nanoparticles

10 g of polybutylcyanoacrylate nanoparticles contain:

| | |
|---|---|
| Active Ingredient | 1.00 g |
| Poloxamer | 0.10 g |
| Butycyanoacrylate | 8.75 g |
| Mannitol | 0.10 g |
| Sodium chloride | 0.05 g |

Polybutylcyanoacrylate nanoparticles are prepared by emulsion polymerization in a water/0.1 N HCI/ethanol mixture as polymerization medium. The nanoparticles in the suspension are finally lyophilized under vacuum.

### Examples

Following examples are used to illustrate procedures for practicing the invention.

### Animals

Male ICR mice weighing 25-30 g were obtained from the Laboratory of Experimental Animals, Riga Stradins University (Riga, Latvia), and housed under standard conditions (21-23 °C, relative humidity 65 % ± 10 %, 12 h light-dark cycle) with unlimited access to standard food (R70 diet, Lactamin AB, Sweden) and water. The experimental procedures were performed in accordance with the guidelines reported in EU Directive 2010/63/EU and in accordance with local laws and policies; all of the procedures were approved by the Latvian Animal Protection Ethical Committee of Food and Veterinary Service in Riga, Latvia. To eliminate systematic differences between the treatment groups, the animals were randomly assigned to their respective experimental groups (12 animals in each group for formalin test and 6 animals in each group for LPS test). To avoid subjective factors in the rating process, all experimental procedures were performed in a blinded fashion.

### Example 1. Effect of (4R)-2-(4-phenyl-2-oxopyrrolidin-1-yl)acetamide on LPS-induced iNOS gene overexpression in brain tissue in mice

The iNOS gene expression was stimulated by a single intraperitoneal (i.p.) injection of LPS at a dose of 20 mg/kg 6 h before the tissue sampling. Control animals received i.p. injection of saline (0.9 % NaCl). To test the acute effect of (4*R*)-2-(4-phenyl-2-oxopyrrolidin-1-yl)acetamide, (4*S*)-2-(4-phenyl-2-oxopyrrolidin-1-yl)acetamide and (4*R*,*S*)-2-(4-phenyl-2-oxopyrrolidin-1-yl)acetamide on LPS-induced overexpression of iNOS gene, LPS was injected simultaneously with (4*R*)-2-(4-phenyl-2-oxopyrrolidin-1-yl)acetamide, (4*S*)-2-(4-phenyl-2-oxopyrrolidin-1-yl)acetamide or (4*R*,*S*)-2-(4-phenyl-2-oxopyrrolidin-1-yl)acetamide at a dose of 50 mg/kg or saline. Mice were decapitated 6 h after the administration of substances.

Brain tissue after sampling were immediately frozen in liquid nitrogen and stored at -80 °C. Total RNA from brain tissue was isolated using the TRI Reagent (Sigma, USA) according to the manufacturer's protocol. Samples were diluted with water at v/v ratio of 1:20 and the quality and quantity of extracted total RNA were examined by measuring the absorbance at 230, 260 and 280 nm with a µQuant™ (BioTek) spectrophotometer. Samples were diluted with water to reach the concentration of 0.5 µg/ml and first-strand cDNA was synthesized using a High Capacity cDNA Reverse Transcription Kit (Applied Biosystems™, USA) following the manufacturer's instructions. Quantitative RT-PCR analysis for iNOS was performed by mixing synthesized cDNA (diluted with water at v/v ratio of 1:10), appropriate primers, and SYBR® Green Master Mix (Applied Biosystems™, USA) and run in the Applied Biosystems Prism 7500 according to the manufacturer's protocol. The transcript levels for the constitutive housekeeping gene product β-actin were quantitatively measured for each sample, and PCR data were reported as the number of transcripts per number of β-actin mRNA molecules.

I.p. administration of LPS after 6 h induced a significant increase in iNOS gene transcription in the mice brain tissue about 12 fold comparing with saline (Fig. 1). Treatment with (4*R*)-2-(4-phenyl-2-oxopyrrolidin-1-yl)acetamide at a dose of 50 mg/kg significantly reduced LPS-induced iNOS gene expression. Unexpectedly treatment with (4*S*)-2-(4-phenyl-2-oxopyrrolidin-1-yl)acetamide and (4*R,S*)-2-(4-phenyl-2-oxopyrrolidin-1-yl)acetamide at a dose of 50 mg/kg did not reduce gene expression comparing with LPS group. Moreover, the efficacy of (4*R*)-2-(4-phenyl-2-oxopyrrolidin-1-yl)acetamide on iNOS gene expression was significantly different compared to (4*S*)-2-(4-phenyl-2-oxopyrrolidin-1-yl)acetamide (Fig. 1).

### Example 2. Effects of (4R)-2-(4-phenyl-2-oxopyrrolidin-1-yl)acetamide on LPS-induced hypothermia in mice

The hypothermia was induced by an i.p. injection of LPS at a dose of 20 mg/kg. Control animals received i.p. injection of saline (0.9 % NaCl). To test the acute effect of (4*R*)-2-(4-phenyl-2-oxopyrrolidin-1-yl)acetamide on LPS-induced hypothermia, LPS was injected simultaneously with (4*R*)-2-(4-phenyl-2-oxopyrrolidin-1-yl)acetamide at a dose of 50 mg/kg or saline. The rectal temperature of animals was measured using a thermometer (Thermalert TH-5, USA) at 5 min before LPS or saline injection and 6 hours after injection.

According to Fig. 2 data, i.p. administration of LPS at a dose 20 mg/kg with saline after 6 h reduced rectal temperature of tested animals. Simultaneous i.p. administration of LPS at a dose 20 mg/kg with (4*R*)-2-(4-phenyl-2-oxopyrrolidin-1-yl)acetamide at a dose of 50 mg/kg significantly inhibited LPS-induced hypothermia.

### Example 3. Effects of (4R)-2-(4-phenyl-2-oxopyrrolidin-1-yl)acetamide on formalin-induced paw licking

Formalin-induced licking paw test was performed as described previously by Zvejniece et al 2006 (Zvejniece L., Muceniece R., Krigere L., Dambrova M., Klusa V. Z. Pharmacol. Biochem. Behav. 2006, 85 (2),287-91). Briefly, mice were gently restrained and 30 µl of formalin solution (1.5 % in saline) was injected s.c. into the plantar surface of the right hind paw, using a microsyringe with a 27-gauge needle. Each mouse was then placed in an individual clear Plexiglas observation chamber (30 x 20 x 30 cm) and the total licking time of hind paw of each mouse was registered and quantified in subsequent 5-minute intervals for 50 minutes. The recording of licking time started immediately (first phase) and lasted for 5 min. The late phase (second phase) started about 15-20 min after formalin injection and lasted up to 35 min.

Pre-treatment with (4*R*)-2-(4-phenyl-2-oxopyrrolidin-1-yl)acetamide at a dose of 50 mg/kg resulted in a significant inhibition of the paw licking response time induced by formalin in II phase of experiment. As can be seen in Fig. 3A and 3B (4*R*)-2-(4-phenyl-2-oxopyrrolidin-1-yl)acetamide at a dose of 50 mg/kg caused a significant reduction by 46 % of the paw licking behavior in phase II (Fig. 3B). However, pre-treatment with (4*R,S*)-2-(4-phenyl-2-oxopyrrolidin-1-yl)acetamide at a dose of 50 mg/kg did not reduce paw-licking time in I and II phase of formalin-induced paw licking test compared to saline treated group (Fig. 4A, 4B).

## Claims

1. A pharmaceutical composition comprising active compound (4*R*)-2-(4-phenyl-2-oxopyrrolidin-1-yl)acetamide or a pharmaceutically acceptable salt, hydrate, solvate, co-crystal or polymorph thereof, for use in the treatment or prevention of a pathological condition, **characterized by** an elevated expression of iNOS gene, which is sepsis or peripheral neuropathy.

2. The pharmaceutical composition for use according to claim 1 for treatment or prevention of sepsis.

3. The pharmaceutical composition for use according to claim 1 for treatment or prevention of peripheral neuropathy.

4. The pharmaceutical composition for use according to claim 1, further comprising a pharmaceutically acceptable carrier or diluent.

5. A pharmaceutical composition for use in the treatment or prevention of sepsis in a living subject in need thereof, wherein the composition comprises a therapeutically effective amount of (4*R*)-2-(4-phenyl-2-oxopyrrolidin-1-yl)acetamide or a pharmaceutically acceptable salt, hydrate, solvate, co-crystal or polymorph thereof.

## Patentansprüche

1. Pharmazeutische Zusammensetzung, umfassend Wirkstoff (4R)-2-(4-Phenyl-2-oxopyrrolidin-1-yl)-acetamid oder ein pharmazeutisch verträgliches Salz, Hydrat, Solvat, Co-Kristall oder Polymorph davon zur Verwendung in der Behandlung oder Vorbeugung eines pathologischen Zustands, der durch eine erhöhte Expression des iNOS-Gens gekennzeichnet ist, welcher Sepsis oder periphere Neuropathie ist.

2. Pharmazeutische Zusammensetzung zur Verwendung nach Anspruch 1 zur Behandlung oder Vorbeugung von Sepsis.

3. Pharmazeutische Zusammensetzung zur Verwendung nach Anspruch 1 zur Behandlung oder Vorbeugung von peripherer Neuropathie.

4. Pharmazeutische Zusammensetzung zur Verwendung nach Anspruch 1, ferner umfassend einen pharmazeutisch verträglichen Träger oder Verdünnungsmittel.

5. Pharmazeutische Zusammensetzung zur Verwendung in der Behandlung oder Vorbeugung von Sepsis in einem lebenden Subjekt, welches diese benötigt, worin die Zusammensetzung eine therapeutisch wirksame Menge von (4R)-2-(4-Phenyl-2-oxopyrrolidin-1-yl)-acetamid oder ein pharmazeutisch verträgliches Salz, Hydrat, Solvat, Co-Kristall oder Polymorph davon umfasst.

## Revendications

1. Composition pharmaceutique comprenant le composé actif (4*R*)-2-(4-phényl-2-oxopyrrolidin-1-yl)-acétamide ou un sel, hydrate, solvate, co-cristal ou polymorphe pharmaceutiquement acceptable de celui-ci, pour une utilisation dans le traitement ou la prévention d'une affection pathologique, **caractérisée par** une expression élevée du gène iNOS, qui est une sepsie ou une neuropathie périphérique.

2. Composition pharmaceutique pour une utilisation selon la revendication 1 pour le traitement ou la prévention d'une sepsie.

3. Composition pharmaceutique pour une utilisation selon la revendication 1 pour le traitement ou la prévention d'une neuropathie périphérique.

4. Composition pharmaceutique pour une utilisation selon la revendication 1, comprenant en outre un support ou un diluant pharmaceutiquement acceptable.

5. Composition pharmaceutique pour une utilisation. dans le traitement ou la prévention d'une sepsie chez un sujet vivant en ayant besoin, dans laquelle la composition comprend une quantité thérapeutiquement efficace de (4*R*)-2-(4-phényl-2-oxopyrrolidin-1-yl)-acétamide ou un sel, hydrate, solvate, co-cristal ou polymorphe pharmaceutiquement acceptable de celui-ci.
